# EUROPEAN PATENT APPLICATION

(11) **EP 4 349 835 A1**
(43) Date of publication of application: **10.04.2024**
(21) Application number: 22815303.7
(22) Date of filing: 01.06.2022
(51) Int. Cl.: C07D 413/14, A61K 31/5377, A61P 35/00

(54) **HYDRATE CRYSTAL FORM OF LAZERTINIB METHANESULFONATE, PREPARATION METHOD THEREFOR AND USE THEREOF**

(30) Priority: 01.06.2021 CN 202110608943
(71) Applicant: Hangzhou Solipharma Co., Ltd., Hangzhou, Zhejiang 310018 (CN)
(72) Inventor: SHENG, Xiaohong, Hangzhou, Zhejiang 310018 (CN); SHENG, Xiaoxia, Hangzhou, Zhejiang 310018 (CN); PENG, Chenyue, Hangzhou, Zhejiang 310018 (CN)
(74) Representative: Müller-Boré & Partner Patentanwälte PartG mbB
(86) International application number: PCT/CN2022/096548
(87) International publication number: WO 2022/253261

(57) **Abstract**

This application relates to the field of chemistry of drugs. It relates to a lazertinib monomethanesulfonate hydrate crystal form, its preparation method thereof and uses. The crystal form of lazertinib monomethanesulfonate hydrate provided in this application has at least one of the following advantageous properties: good stability, high solubility, good dissolution, low hygroscopicity, uniform particle size distribution, favorable morphology, good flowability, and high crystallinity. These characteristics contribute to stable storage, preventing degradation, increased impurities, changes in appearance, and crystallization during development and storage. The preparation method is simple and reliable, offering significant development value.

## Description

### CROSS-REFERENCE TO RELATED APPLICATIONS

This application claims all the benefits of the Chinese patent application No. 202110608943.2 filed on June 1, 2021 with the National Intellectual Property Administration of the People's Republic of China, of which the entire contents are incorporated herein by reference.

### TECHNICAL FIELD

The present application relates to the field of chemistry field of medicine. In particular, the present application relates to a hydrate crystal form of lazertinib mesylate, preparation method therefor and use therefor.

### BACKGROUND

Polymorph or polymorphism is a particular property of certain molecule and molecular composition. Different crystalline forms of compounds arise from different molecular packing in crystal lattices, and different crystalline forms have different crystal structures and different physical properties, such as solubility, stability, thermal property, mechanical property, purification ability , X-ray diffraction pattern, infrared absorption spectroscopy, Raman spectroscopy, solid state nuclear magnetic resonance, etc.

The discoverying novel crystal forms of pharmaceutically active ingredients (including anhydrates, hydrates, and solvates, etc.) may lead to lead to materials with enhanced processing advantages or improved physicochemical properties. These properties can include better bioavailability, better storage stability, better processibility, and ease of purification, or serving as intermediate forms that facilitate the transformation into other crystal forms. Some specific crystal forms of active pharmaceutical ingredients can also contribute to the improvement of drug performance. This expands the range of available material forms in formulation science, such as improving dissolution rates, extending shelf life, and facilitating easier processing.

Lazertinib (trade name LECLAZA) is a third-generation pidermal growth factor receptor (EGFR) tyrosine kinase inhibitor (TKI) developed by Yuhan, targeting the T790M mutation and activating the EGFR mutation while preserving the wild-type EGFR. Lazertinib can be used as a drug to inhibit the activity of protein kinase-mediated disorders, especially those involving the inhibition of EGFR with one or more mutations compared to the wild-type EGFR. Lazertinib's IUPAC name is N-[5-[[4-[4-[(dimethylamino)methyl]-3-phenylpyrazol-1-yl]pyrimidin-2-yl]amino]-4-methoxy-2-morpholin-4-ylphenyl]prop-2-enamide, with a structural formula shown in formula (I). It is primarily used in the clinics for the treatment of metastatic non-small cell lung cancer. with the drug being used in the form of mono-methanesulfonate salt of the compound (I),

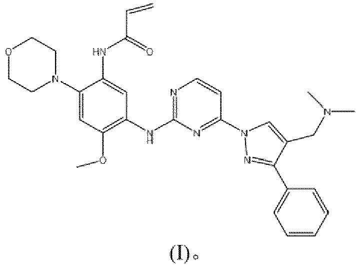

Different solid forms of small molecule chemical drugs, namely different crystalline forms, can result in differences in solubility and stability, thereby affecting drug absorption and bioavailability, and may even lead to differences in clinical efficacy.CN110869367A reported the crystalline form (I) of lazertinib mono-mesylate (hereinafter referred to as "Form I"). The applicant attempted to prepare the material according to the method in Example 1 of the patent application, and but encountered incomplete reaction. Despite successfully obtaining Form I by improving the preparation method, it was found that during accelerated stability studies, the sample exhibited discoloration, increased impurities, and poor stability, making it unsuitable for long-term storage.

Therefore, there is still a need in this field for a new crystal form of high solubility, stability, and suitable for storage, for the monomethanesulfonate salt of lazertinib with high pharmaceutical value. This would facilitate the development, production, and storage of lazertinib.

### SUMMARY OF THE INVENTION

The purpose of this application is to provide a hydrate crystal form, its preparation method, and use of lazertinib mono-mesylate (hereinafter referred to as lazertinib mesylate). The hydrate crystal form of lazertini mesylate in this application has at least one of the following advantages: good stability, good solubility, good dissolution, low hygroscopicity, uniform particle size distribution, good morphology, good flowability, good crystallinity, stable for storage, avoiding drug degradation, increased impurity, changes in appearance, and form transformation during development and storage. The preparation method is simple and reliable, demonstrating significant development value.

One aspect of this application is to provide a hydrate form A (hereinafter referred to as Form A) of lazertinib mesylate, with a structure of lazertinib as shown in formula (I): 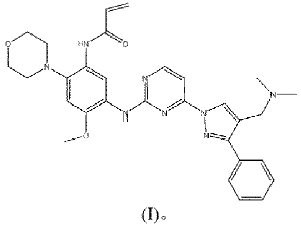 wherein using Cu-Kα radiation, the X-ray powder diffraction (XRPD) pattern of Form A is characterized by comprising at least three characteristic peaks at 5.68°±0.2°, 11.74±0.2°, 15.60°±0.2°, 21.61°±0.2°, or 22.71°±0.2° 2θ.

In a preferred embodiment of this application, the X-ray powder diffraction (XRPD) pattern of Form A further comprises characteristic peaks at least one peak at 8.62±0.2°, 10.50°±0.2°, 14.50±0.2°, 16.57°±0.2°, or 17.21±0.2° 2θ.

In a preferred embodiment of this application, the X-ray powder diffraction (XRPD) pattern of Form A further comprises characteristic peaks at least one peak at 11.24°±0.2°, 13.30±0.2°, 18.17±0.2°, 20.03±0.2°, 24.63°±0.2°, or 28.53±0.2° 2θ.

In a preferred embodiment of this application, the X-ray powder diffraction (XRPD) pattern of Form A has characteristic peaks at the following position (2θ) shown in the table below:

| 2θ±0.2° |
|---|
| 5.68 |
| 8.62 |
| 10.50 |
| 11.24 |
| 11.74 |
| 13.30 |
| 14.51 |
| 15.60 |
| 16.57 |
| 17.21 |
| 17.91 |
| 18.17 |
| 19.55 |
| 20.03 |
| 20.99 |
| 21.61 |
| 22.71 |
| 23.45 |
| 24.63 |
| 25.61 |
| 25.91 |
| 26.57 |
| 27.41 |
| 28.53 |
| 29.61 |

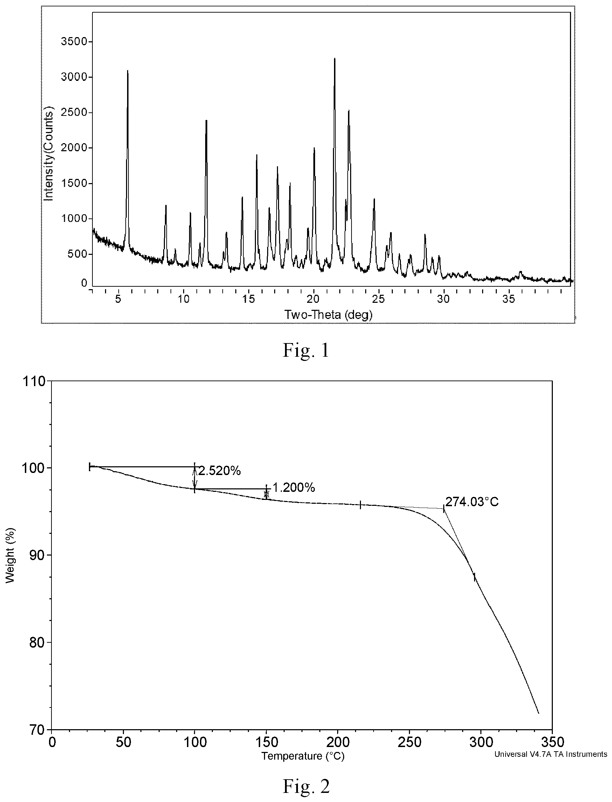

Without imposing restrictions, Form A exhibits an X-ray powder diffraction (XRPD) pattern essentially as depicted in Figure 1.

In another aspect of the present application, the Thermogravimetric Analysis (TGA) thermogram of Form A may exhibit a two-stage weight loss, but the application is not limited to this configuration.

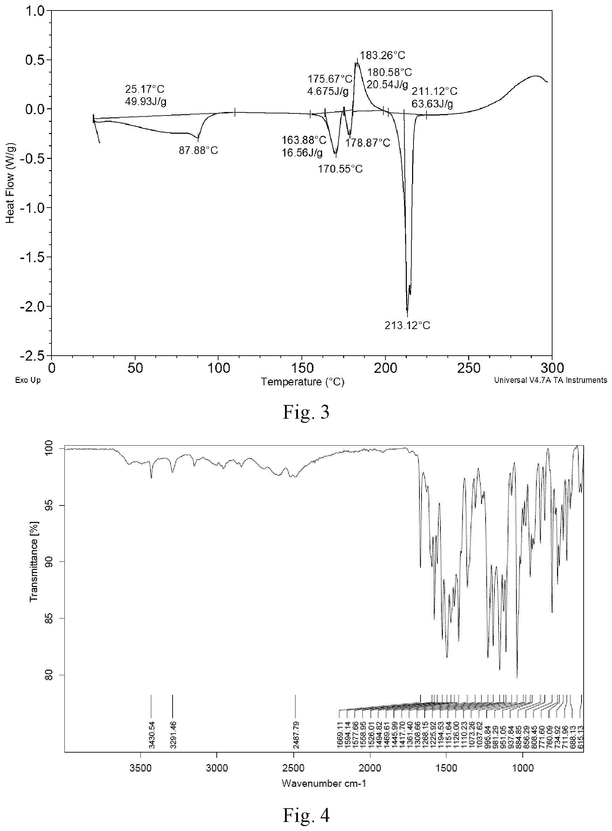

Non-restrictively, in a specific embodiment, the Thermogravimetric Analysis (TGA) thermogram of Form A essentially appears as shown in Figure 2. Non-restrictively, in a specific embodiment, the Differential Scanning Calorimetry (DSC) thermogram of Form A essentially appears as shown in Figure 3. In the preferred embodiment of the present application, the Fourier Transform Infrared (FT-IR) spectrum of Form A exhibits at least one band at 760.06 cm⁻¹ ± 2 cm⁻¹, 1151.59 cm⁻¹ ± 2 cm⁻¹, 2487.87 cm⁻¹ ± 2 cm⁻¹, or 3291.47 cm⁻¹ ± 2 cm⁻¹.

In the preferred embodiment of the present application, the Fourier Transform Infrared (FT-IR) spectrum of Form A also exhibits at least one band at 1669.10 cm⁻¹ ± 2 cm⁻¹, 1361.34 cm⁻¹ ± 2 cm⁻¹, 1225.88 cm⁻¹ ± 2 cm⁻¹, 1194.47 cm⁻¹ ± 2 cm⁻¹, 110.26 ± 2 cm⁻¹, 1037.52 cm⁻¹ ± 2 cm⁻¹, 808.41 cm⁻¹ ± 2 cm⁻¹, or 771.53 cm⁻¹ ± 2 cm⁻¹.

Non-restrictively, the Fourier Transform Infrared (FT-IR) spectrum of Form A essentially appears as shown in Figure 4.

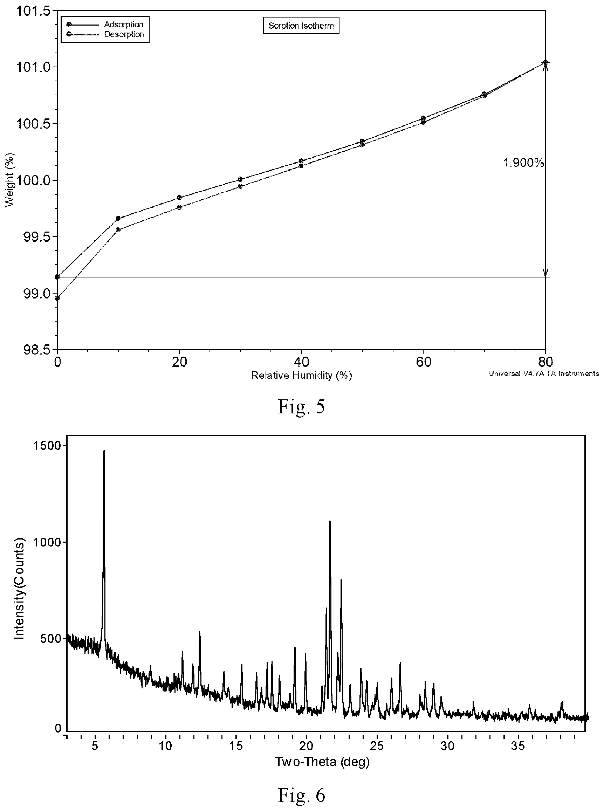

Non-restrictively, the Dynamic Vapor Sorption (DVS) plot of Form A essentially appears as shown in Figure 5.

Another aspect of the present application is to provide a preparation method for the hydrate Form A of lazertinib mesylate. The method includes any one of the following methods:
1) Dissolve lazertinib and methanesulfonic acid separately in dichloromethane to form a clear solution of lazertinib and a clear solution of methanesulfonic acid. Mix the two solutions, precipitate the resulting solid, centrifuge, and dry to obtain the hydrate Form A of lazertinib mesylate. The dissolution of lazertinib is carried out at an elevated temperature, and the molar ratio of lazertinib to methanesulfonic acid is in the range about 1:1 to about 1.2.

Preferably, the elevated temperature is 60°C or higher; furthermore, it is in the range of 65-85°C.

Preferably, the mixing of the clear solutions involves adding the methanesulfonic acid solution into the lazertinib solution.

Preferably, the drying process is conducted through room temperature vacuum drying.

2) Form solution 1 by dissolving lazertinib mesylate form B (Form B) in solvent 1. Then, add solution 1 to solvent 2, stir, precipitate solid material, centrifuge, and dry to obtain the hydrate Form A of lazertinib mesylate. In this process, solvent 1 and solvent 2 are mutually miscible solvents.

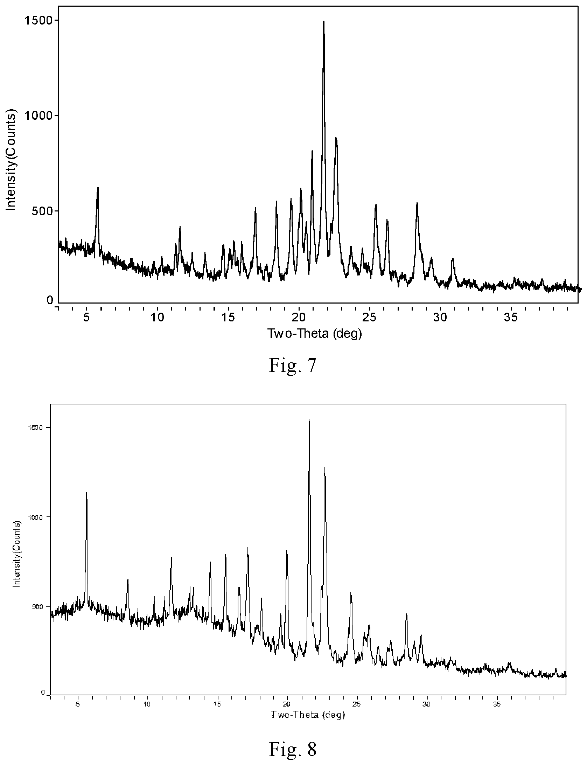

Preferably, Form B exhibits an X-ray powder diffraction (XRPD) pattern essentially as shown in Figure 7.

Preferably, solvent 1 is selected from trichloromethane.

Preferably, solvent 2 is selected from ethyl ether or acetonitrile.

Preferably, the volume ratio of solvent 1 to solvent 2 is less than 1:5; more preferably, less than 1:7.

Preferably, drying is conducted at room temperature. 3) Dry lazertinib mesylate Form B at 40-90°C to obtain the hydrate Form A of lazertinib mesylate.

Preferably, the drying temperature is in the range about 40 to about 50°C.

The hydrate Form A of lazertinib mesylate described in this application has the following beneficial effects:
1) The hydrate Form A of lazertinib mesylate in this application exhibits excellent stability. Under accelerated sealed conditions (40°C, 75%RH, with 5 mL vial packed in a self-sealing bag), it remains stable for at least 1 month with no significant changes in crystal form, melting point, assay value, and color before and after storage. Additionally, under accelerated open conditions (40°C, 75%RH, open), it can remain stable for at least 21 days with no observable changes in crystal form, assay value, and color before and after storage. In contrast, the prior art's lazertinib mesylate Form I, under accelerated open conditions (40°C, 75%RH, open) for 21 days, experiences a significant decrease in assay value, an increase in impurities, and a color change from yellow to pink.
2) The hydrate Form A of lazertinib mesylate in this application demonstrates excellent solubility compared to the prior art's lazertinib mesylate Form I. Form A exhibits unexpectedly superior solubility, with equilibrium solubility greater than 60 mg/mL in pH 4.0 buffer, water, and pH 1.2 buffer. In contrast, under the same test conditions, the solubility of Form I in the pH 4.0 buffer, water, and pH 1.2 buffer, as tested in CN110869367A Example 1, is 20.9 mg/mL, 21.6 mg/mL, and 14.9 mg/mL, respectively-significantly lower than the solubility of form A in this application.

Additionally, Form A in this application unexpectedly exhibits excellent instantaneous solubility, with instantaneous solubility greater than 50 mg/mL in pH 4.0 buffer and water, and greater than 80 mg/mL in pH 1.2 buffer. This is advantageous for achieving the desired drug bioavailability and efficacy, meeting pharmaceutical requirements.

3) The hydrate Form A of lazertinib mesylate in this application exhibits good dissolution characteristics when formulated into tablets, meeting pharmaceutical requirements. Under the same experimental conditions, tablets made from Form A and tablets made from Form I both achieve 100% dissolution within 10 minutes in a pH 1.2 solution. Therefore, the dissolution rate of tablets made from Form A is comparable to that reported in the prior art's patent for tablets made from Form I.

4) The preparation method for the hydrate Form A of lazertinib mesylate in this application is simple, exhibits high repeatability, and holds promising potential for industrialization.

Another aspect of the present application provides a pharmaceutical composition of lazertinib. The composition comprises hydrate Form A of lazertinib mesylate and at least one pharmaceutically acceptable carrier.

Another aspect of the present application is to provide a formulation prepared from the aforementioned lazertinib pharmaceutical composition. The formulation may take various forms, including but not limited to oral solid dosage forms, topical formulations, and injectable formulations.

In a preferred embodiment of the present application, the formulation form is selected from tablets, capsules, pills, suppositories, granules, fine granules, powders sustained-release formulations, immediate-release formulations, solutions, suspensions, elixirs, aerosols, etc.

In a preferred embodiment of the present application, the formulation form is a tablet.

The pharmaceutically acceptable carriers are commonly used excipients in the field, including but not limited to binders, adhesives, surfactants, diluents, anti-adherents, hydrophilic or hydrophobic polymers, and stabilizers, disintegrants, antioxidants, defoaming agents, fillers, glidants/lubricants, adsorbents, preservatives, plasticizers, sweeteners, and any two or more of these carriers.

In the preferred embodiment of the present application, when the formulation is an oral solid dosage form, the filler or diluent is selected from lactose, microcrystalline cellulose, starch, pregelatinized starch, calcium sulfate, calcium hydrogen phosphate, and calcium carbonate, or any combination thereof; the disintegrant is selected from sodium carboxymethyl starch, croscarmellose sodium, low-substituted hydroxypropyl cellulose, and crospovidone, or any combination thereof; the lubricant/glidant is selected from magnesium stearate, talc, and micropowder silica gel, or any combination thereof.

Furthermore, the pharmaceutical composition may also comprise one or more pH adjusting agents or buffers. For example, acids such as acetic acid, boric acid, citric acid, fumaric acid, maleic acid, tartaric acid, malic acid, lactic acid, phosphoric acid and hydrochloric acid, or any combination thereof; or bases, such as sodium hydroxide, sodium phosphate, sodium borate, sodium citrate, sodium acetate, sodium lactate, tris or any combination thereof; Or buffers such as citrate/glucose, sodium bicarbonate, ammonium chloride, or a like. Such buffers, when used as a base, may have counterions other than sodium, such as potassium, magnesium, calcium, ammonium, and other counterions; and the necessary amount of such acids, bases, and buffers to maintain the pH of the components within an acceptable range can be present in the form of a solution or solid.

Another aspect of the present application is to provide a use of hydrate Form A of lazertinib mesylate or the pharmaceutical composition thereof in the preparation of drugs for treating protein kinase-mediated diseases.

Preferably, the protein kinase-mediated disease is cancer.

Preferably, the cancer is non-small cell lung cancer, metastatic non-small cell lung cancer, brain metastatic non-small cell lung cancer, EGFR-positive non-small cell lung cancer, or non-squamous non-small cell lung cancer.

Another aspect of the present application is to provide a use of the hydrate Form A of lazertinib mesylate or the pharmaceutical composition thereof in the preparation of drugs for inhibiting the activity of EGFR with at least one mutation compared to wild-type EGFR.

Another aspect of the present application is to provide a method for treating a protein kinase-mediated disease. The method comprises administering to a patient an effective amount of hydrate Form A of lazertinib mesylate or the pharmaceutical composition thereof.

Preferably, the protein kinase-mediated disease is cancer.

Preferably, the cancer is non-small cell lung cancer, metastatic non-small cell lung cancer, brain metastatic non-small cell lung cancer, EGFR-positive non-small cell lung cancer, or non-squamous non-small cell lung cancer.

Another aspect of the present application is to provide a method for inhibiting the activity of EGFR with at least one mutation compared to wild-type EGFR. The method comprises administering to a patient an effective amount of the hydrate Form A of lazertinib mesylate or the pharmaceutical composition thereof.

Preferably, the effective amount of the hydrate Form A of lazertinib mesylate in one of the applications of the present application is in the range about 0.001 to about 10 mg/kg, more preferably in the range of about 0.005 to about 5 mg/kg.

Preferably, the method can be administered once a day, twice a day, three times a day, or more; a single dose can range from in the range about 0.1 mg to about 500 mg/kg/day, with the specific dose determined based on the patient's condition.

Preferably, the method involves once-daily administration.

Preferably, the single dose is for oral administration of hydrate Forms A of lazertinib mesylate at 10, 20, 40, 60, 80, 100, 160, 240, 320, or 400 mg; more preferably, 240 mg.

Another aspect of the present application is to provide a combination use of hydrate Form A of lazertinib mesylate or its pharmaceutical composition with other drugs.

Preferably, the other drugs are selected from midazolam, rosuvastatin, metformin, amitriptyline, itraconazole, rifampicin, gefitinib, pemetrexed, carboplatin, Ami-HC-CF, and Ami-HC.

The term "channel hydrate" is used to describe stoichiometric or non-stoichiometric hydrates in crystal structures that have one-dimensional channels or two-dimensional planes filled with water. In non-stoichiometric hydrates, the amount of water in the lattice can vary with the surrounding atmospheric water vapor pressure and temperature.

Unless otherwise specified:
The experimental operating temperature generally refers to room temperature, and "room temperature" refers to a temperature in the range about 10°C to about 30°C.

When considered by those skilled in the art, the term "approximately" typically means falling within an acceptable range of average value error standards.

The XRD (X-ray diffraction), TGA (thermogravimetric analysis), and water adsorption behavior of the hydrate Form A of Lazertinib meslyate indicate that this crystal form gradually adsorbs and desorbs varying amounts of water in response to changes in humidity. Such water characteristics are typical of channel hydrates (variable hydrates). In some cases, Form A may be referred to as a non-stoichiometric hydrate or a non-stoichiometric channel hydrate. Additionally, the crystal form undergoes a transformation after losing another portion of water at a high temperature (approximately 150°C).

"Stirring" may be carried out by conventional methods in the art. For example, stirring includes magnetic stirring, mechanical stirring, and the like. and the stirring speed is 50-1800 rpm, preferably 300-900 rpm.

"Separation" may be carried out by conventional methods in the art, such as centrifugation or filtration. "Separation" may employ conventional methods in the art, such as centrifugation or filtration. Preferred vacuum filtering, generally at a pressure less than atmospheric pressure for filtration, preferably less than 0.09MPa.

"Drying" can be carried out by conventional techniques in the art, such as normal temperature drying, blast drying or reduced pressure drying. Reduced pressure or atmospheric pressure may be used. Reduced pressure, preferably with a pressure less than 0.09MPa. The drying apparatus and method are not limited, and may be a fume hood, a blast oven, a spray dryer, a fluidized bed drying, or a vacuum oven, which may also be carried out under reduced or non-reduced pressure, Reduced pressure, preferably with a pressure less than 0.09MPa.

Unless otherwise specified, the ratios involved in this application, between solid and liquid, are mass-volume ratios, and between liquids and liquids, are volume ratios.

### DESCRIPTION OF THE DRAWINGS

Fig. 1 The XRPD pattern of the sample in Example 1-1 (hydrate Form A of lazertinib mesylate).
Fig. 2 The TGA thermogram of the sample in Example 1-1.
Fig. 3 The DSC thermogram of the sample in Example 1-1.
Fig. 4 The FTIR spectrum of the sample in Example 1-1.
Fig. 5 The DVS plot of the sample in Example 1-1.
Fig. 6 The XRPD pattern of the sample in Preparation Example 1 (Form I of lazertinib mesylate).
Fig. 7 The XRPD pattern of the sample in Preparation Example 2 (Form B of lazertinib mesylate).
Fig. 8 The XRPD pattern of the sample in Example 1-2 (hydrate Form A of lazertinib mesylate).
Fig. 9 The TGA thermogram of the sample in Example 1-2.
Fig. 10 The XRPD pattern of the sample in Example 1-3 (hydrate Form A of lazertinib mesylate).
Fig. 11 The TGA thermogram of the sample in Example 1-3.
Fig. 12 The XRPD overlay of hydrate Form A of lazertinib mesylate before and after accelerated closed-vial conditions.
Fig. 13 The XRPD overlay of hydrate Form A of lazertinib mesylate before and after accelerated open-vial conditions
Fig. 14 The color comparison of hydrate Form A of lazertinib mesylate before and after accelerated open-vial conditions.

### DETAILED DESCRIPTION

The technical implementations of the present application are described in detail below with reference to the accompanying drawings and embodiments. However, this does not limit the scope of this application to the specific embodiments described.

In this application, X-ray powder diffraction (XRPD) data were collected using a BrukerD8 Advance diffractometer; parameters are as follows: Cu radiation; wavelength: 1.54Å; Current voltage: 40KV, 40mA; Scan range: 3~40°2θ.

In this application, the thermogravimetric analysis (TGA) data were collected using a TA Instruments Q500 TGA; the parameters were as follows: high resolution mode; heating rate: 10°C/min; gas: N2; sample pan: platinum crucible.

In this application, Differential Thermal Analysis (DSC) data were obtained using a TA Instruments Q200 DSC; parameters were as follows: ramp rate: 10°C/min; gas: N₂; sample pan: aluminum pan with lid.

In this application, dynamic moisture adsorption analysis (DVS) data and isothermal adsorption analysis data were obtained using a TA Instruments Q5000 TGA; parameters are as follows: temperature: 25°C; relative humidity range: 0%RH-80%RH; dm/dt =0.001%/min; equilibration time: 90min; gas: N₂; sample pan: platinum pan.

In this application, Fourier transform infrared spectroscopy (FT-IR) data were collected using a Bruker Tensor 27; the parameters were as follows: ATR method, collection range 600cm⁻¹-4000cm⁻¹, resolution 4cm⁻¹.

In this application, proton nuclear magnetic resonance data (¹H NMR) was acquired using a Bruker Ascend 500 MHz nuclear magnetic resonance spectrometer. An appropriate amount of sample was weighed and dissolved in approximately 0.5 mL of deuterated dimethyl sulfoxide (DMSO-d₆) for analysis in the NMR sample tube.

In this application, the detection parameters for high-performance liquid chromatography (HPLC) for assay, solubility, and dissolution data are as follows:

| | |
|---|---|
| Column | Titank-C18 150 ×4.6mm, 3 µm |
| Mobile phase | Phase A: 0.1% Trifluoroacetic Acid |
| | Phase B: Methanol |

In the following embodiments, experimental methods without specific conditions are conducted using standard procedures and conditions, or as instructed in commercial manuals. Unless otherwise specified, the reagents and materials used in this application are commercially available.

In this application, the free form of lazertinib in the starting materials can be obtained commercially or prepared using methods mentioned in existing technologies, such as the method described in WO2016060443A2.

### Preparation Example 1

### Preparation of Lazertinib Methanesulfonate Form I

Take approximately 5 g of the free form of lazertinib and add it to 35 mL of tetrahydrofuran (THF). Stir the mixture at 70°C to form a lazertinib solution. Take about 1472 mg of methanesulfonic acid (methanesulfonate), add it to 10 mL of THF, and mix to form a methanesulfonate solution.

Add 6.8 mL of the methanesulfonate solution to the lazertinib solution. Stir the mixture at room temperature for 2 hours to precipitate solid. Then, add 35 mL of n-heptane while continuing to stir. Collect the solid obtained.

Place the collected solid under vacuum drying overnight at 30°C to obtain lazertinib methanesulfonate Form I. Its XRPD pattern is shown in Fig. 6.

### Preparation Example 2

### Preparation of Lazertinib Methanesulfonate Form B

Take approximately 50 mg of lazertinib methanesulfonate Form I obtained in Preparation Example 1. Add 1 mL of dichloromethane to the Crystal Form I and stir the slurry at room temperature overnight. Centrifuge the mixture, let it dry at room temperature, and obtain lazertinib methanesulfonate Form B. Its XRPD pattern is shown in Fig. 7.

### Example 1-1

### Preparation of Lazertinib Methanesulfonate Hydrate Form A

Take approximately 110 mg of free-form lazertinib, add it to 0.4 mL of dichloromethane, and dissolve it at 80°C to form a lazertinib solution. Take 149.48 mg of methanesulfonic acid, dissolve it in 1 mL of dichloromethane to form a methanesulfonic acid solution.

Add 0.14 mL of the methanesulfonic acid solution to the lazertinib solution. Solids precipitate at room temperature. Centrifuge the mixture, collect the solid obtained, and dry it under vacuum at room temperature to obtain lazertinib methanesulfonate hydrate Form A.

Its XRPD pattern is shown in the table below:

| 2θ° | I% |
|---|---|
| 5.68 | 86.9 |
| 8.62 | 27.4 |
| 10.50 | 25.6 |
| 11.24 | 11.0 |
| 11.74 | 70.6 |
| 13.30 | 17.7 |
| 14.51 | 34.9 |
| 15.60 | 55.4 |
| 16.57 | 29.5 |
| 17.21 | 48.4 |
| 17.91 | 12.5 |
| 18.17 | 40.3 |
| 19.55 | 19.2 |
| 20.03 | 58.5 |
| 20.99 | 5.0 |
| 21.61 | 100 |
| 22.71 | 74.5 |
| 23.45 | 3.0 |
| 24.63 | 34.7 |
| 25.61 | 11.7 |
| 25.91 | 19.0 |
| 26.57 | 8.9 |
| 27.41 | 10.2 |
| 28.53 | 19.5 |
| 29.61 | 9.8 |

Its XRPD pattern is shown in Fig. 1.

According to the nuclear magnetic resonance (NMR) data, the molar ratio of methanesulfonic acid to lazertinib can be calculated as 1: 1.

Its TGA thermogram is shown in Fig.2, demonstrating a hydrate.

Its DSC thermogram is shown in Fig. 3.

Its FTIR spectrum is shown in Fig. 4.

Its DVS plot is shown in Fig. 5.

Its variable-temperature XRPD pattern shows when heated up to 100°C, its crystal form remains unchanged.

### Example 1-2

Preparation of Lazertinib Methanesulfonate Hydrate Form A Take approximately 150 mg of lazertinib methanesulfonate Form B obtained in Preparation Example 2. Place Form B under vacuum drying at 50°C.

Obtain lazertinib methanesulfonate hydrate Form A.

Its XRPD data is shown in the table below:

| 2θ° | I% |
|---|---|
| 5.62 | 50.6 |
| 8.57 | 17.5 |
| 10.47 | 10.6 |
| 11.20 | 9.5 |
| 11.69 | 26.1 |
| 13.26 | 13.3 |
| 14.46 | 25.6 |
| 15.53 | 31.3 |
| 16.53 | 19.8 |
| 17.16 | 38.7 |
| 17.85 | 7.5 |
| 18.13 | 19.7 |
| 18.98 | 5.3 |
| 19.51 | 16.6 |
| 19.97 | 44.9 |
| 20.82 | 5.9 |
| 21.53 | 100 |
| 22.67 | 79.8 |
| 23.41 | 4.0 |
| 24.55 | 29.7 |
| 25.49 | 12.6 |
| 25.85 | 16.1 |
| 26.51 | 7.4 |
| 27.37 | 10.1 |
| 28.51 | 20.7 |
| 29.55 | 11.7 |

Its XRPD pattern is shown in Fig. 8.

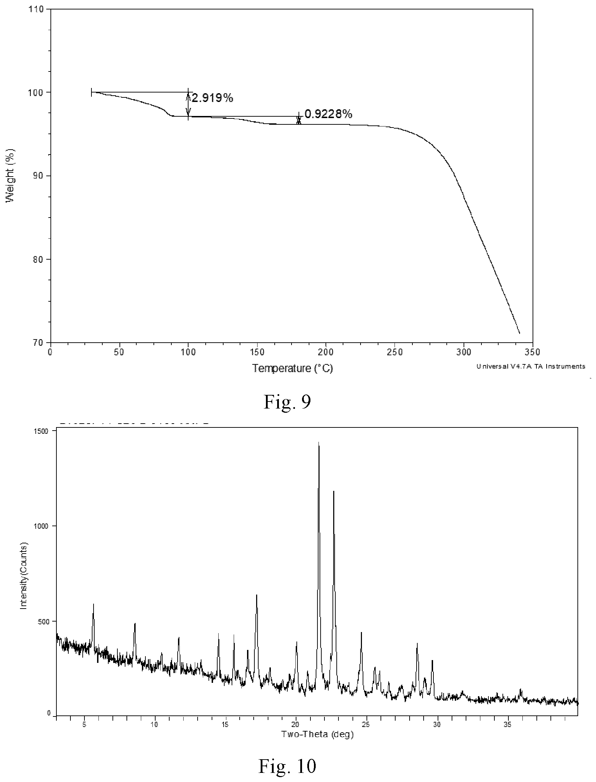

Its TGA thermogram is shown in Fig. 9.

### Example 1-3

### Preparation of Lazertinib Methanesulfonate Hydrate Form A

Take approximately 30 mg of lazertinib methanesulfonate Form B obtained in Preparation Example 2. Add 0.4 mL of chloroform to dissolve Form B, forming a methanesulfonate solution. Add the methanesulfonate solution into 3 mL of ethyl ether, stir immediately to precipitate the solid. Centrifuge the mixture, collect the solid obtained, and air-dry at room temperature to obtain lazertinib methanesulfonate hydrate Form A.

Its XRPD data is shown in the table below:

| 2θ° | I% |
|---|---|
| 5.64 | 19.8 |
| 8.59 | 15.2 |
| 10.48 | 6.8 |
| 11.19 | 5.3 |
| 11.70 | 13.9 |
| 13.24 | 5.4 |
| 14.49 | 18.4 |
| 15.57 | 19.4 |
| 16.55 | 14.3 |
| 17.19 | 37.0 |
| 17.88 | 5.2 |
| 18.14 | 8.0 |
| 19.51 | 6.4 |
| 20.03 | 19.3 |
| 20.81 | 8.3 |
| 21.59 | 100 |
| 22.65 | 80.0 |
| 23.42 | 2.9 |
| 24.61 | 25.2 |
| 25.57 | 11.3 |
| 25.91 | 10.4 |
| 26.55 | 5.8 |
| 27.45 | 5.4 |
| 28.53 | 20.6 |
| 29.61 | 14 |

Its XRPD pattern is shown in Fig. 10.

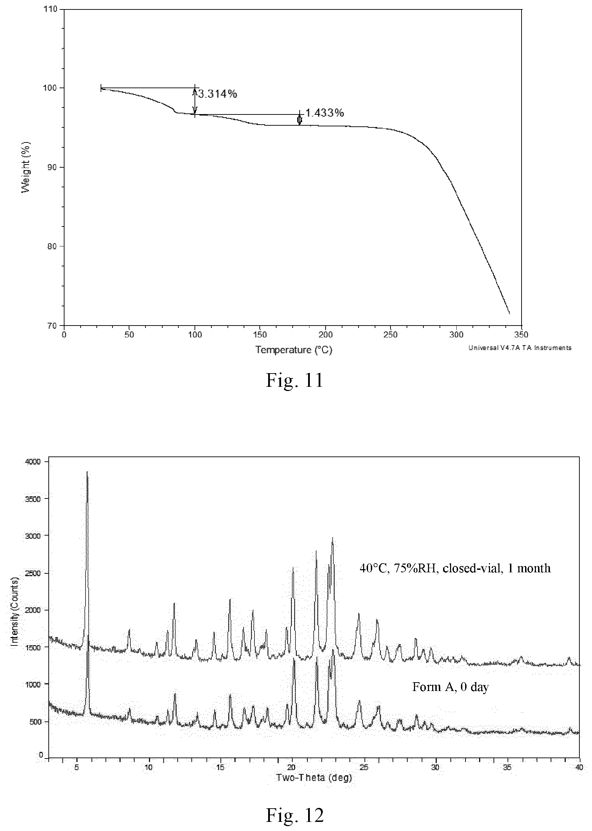

Its TGA thermogram is shown in Fig. 11.

### Example 1-4

### Preparation of Hydrate Form A of Lazertinib

Replace the ethyl ether in Example 1-3 with acetonitrile under the same conditions to prepare lazertinib methanesulfonate hydrate Form A.

### Experiment 1

### Accelerated Closed-Vial Stability Study

According to the testing method in Example 3 of CN110869367A, an appropriate amount of lazertinib methanesulfonate hydrate Form A sample prepared in Example 1-1 of this application was taken. The sample was placed under accelerated closed-vial conditions (40°C, 75% RH, and sealed in a 5 mL vial within a self-sealing bag) to assess the stability of Form A. The results indicate that Form A remains unchanged in its crystal form, and its color remains consistent (white) before and after placement. Additionally, there is minimal change in assay value, demonstrating excellent stability. (See Fig. 12).

### Experiment 2

### Accelerated Open-Vial Stability Study

An appropriate amount of lazertinib methanesulfonate hydrate Form A sample prepared in Example 1-1 of this application and lazertinib methanesulfonate Form I sample prepared in Preparation Example 1 of this application were taken, respectively. The samples were placed under accelerated open-vial conditions (40°C, 75% RH, and open vial) to assess the stability of both samples. The results are shown in the table below.

**Table 1 The Stability of Form A and Form I at Accelerated Open-Vial conditions**

| | | | | | |
|---|---|---|---|---|---|
| Accelerated Open-Vial Conditions (40°C, 75%RH) | Form A | Testing day | XRPD | Assay (%) | Color |
| | | 0 day | Form A | 98.79 | white |
| | | 21 days | Form A | 98.44 | white |
| | Form I | 0 day | Form I | 98.73 | yellow |
| | | 21 days | Form I | 70.35 | pink |

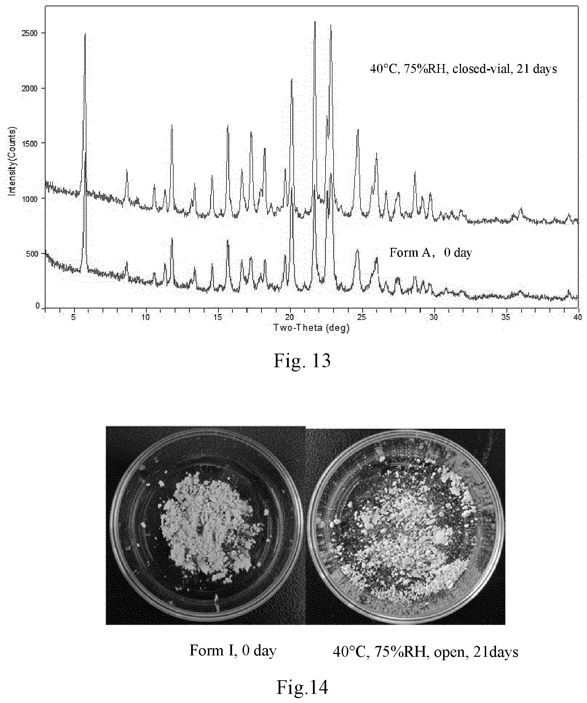

Results indicate that: lazertinib methanesulfonate Hydrate Form A, under accelerated open-vial conditions (40°C, 75%RH, open), remains stable for at least 21 days. The crystal form (Fig. 13) remains unchanged, with no significant variation in assay value and color. In contrast, lazertinib methanesulfonate Form I, after 21 days, exhibits a significant decrease in assay value, increased impurities, and a color change from yellow to pink (Fig. 14).

### Experiment 3

### Solubility of Form A

Following the dissolution test parameters in CN110869367A for Example 1, the dissolution test of lazertinib methanesulfonate Hydrate Form A from this application was conducted. The results are shown in the table below:

**Table 2 Solubility of Form A**

| | Sample | Testing time | pH 4.0 Buffer | Water | pH 1.2 Buffer |
|---|---|---|---|---|---|
| Solubility (mg/mL) | Form A | 4 hrs | 67.9 | 70.7 | > 60 |
| | Form A | 12 hrs | 67.0 | 69.4 | > 60 |

Results show that Lazertinib methanesulfonate Hydrate Form A from this application meets pharmaceutical requirements for equilibrium solubility in pH 4.0 buffer, water, and pH 1.2 buffer. Form A unexpectedly exhibits excellent solubility, with values exceeding 60 mg/mL in pH 4.0 buffer, water, and pH 1.2 buffer, which is advantageous for achieving the desired bioavailablity, meeting pharmaceutical requirements.

### Experiment 4

### Instantaneous Solubility of Form A

Successively taking 50 mg and 80 mg of Lazertinib Methanesulfonate Hydrate Form A from this application, and adding them to 1 ml of different media, including pH 4.0 buffer, water, and pH 1.2 buffer (prepared according to the method of Example 1 in CN110869367A), briefly sonicated, and immediately observing whether the solution is clear. The results are shown in the table below:

**Table 3 Instantaneous Solubility of Form A**

| Media | pH 4.0 Buffer | | Water | | pH 1.2 Buffer | |
|---|---|---|---|---|---|---|
| Testing Sample | Observation | Solubility (mg/mL) | Observation | Solubility (mg/mL) | Observation | Solubility (mg/mL) |
| 50mg | Clear | >50 | Clear | >50 | Clear | >50 |
| 80mg | Cloudy | / | Cloudy | / | Clear | >80 |

The results indicate that the instantaneous solubility of the hydrated Form A of lazertinib mesylate in pH 4.0 buffer, water, and pH 1.2 buffer meets pharmaceutical requirements. Form A of the present application unexpectedly exhibits excellent instantaneous solubility, with values greater than 50 mg/mL in pH 4.0 buffer and water, and greater than 80 mg/mL in pH 1.2 buffer. This is advantageous for achieving the desired bioavailability, meeting pharmaceutical requirements.

### Example 1-5

### Preparation of Tablets

According to the formulation in the table below, mix the API (Form A obtained from Example 1-1), excipients, and disintegrant using a mixer. Then, separately mix the lubricant, and after compression, obtain tablets.

**Table 4 Formulation of Tablets**

| | | Tablet 1 | Tablet 2 | Tablet 3 | Tablet 4 | Tablet 5 |
|---|---|---|---|---|---|---|
| | Ingredient | Each Tablet (mg) | Each Tablet (mg) | Each Tablet (mg) | Each Tablet (mg) | Each Tablet (mg) |
| API | Form A | 12.22 | 48.88 | 48.88 | 97.76 | 122.19 |
| Filler | Microcrystalline cellulose | 65.56 | 65.51 | 65.11 | 67.14 | 67.66 |
| | D-mannitol | 65.25 | 65.23 | 65.24 | 66.12 | 67.13 |
| Disintegrant | Dross-linked sodium carboxymethyl | 6.37 | 6.06 | 6.10 | 6.20 | 5.99 |
| Lubricant | Magnesium stearate | 2.41 | 2.13 | 2.36 | 2.20 | 2.00 |
| | Total Content | 151.03 | 186.13 | 185.68 | 239.32 | 260.77 |
| Note: 12.22 mg of Form A is equivalent to 10 mg of free compound, 48.88 mg of Form A is equivalent to 40 mg of free compound, 97.76 mg of Form A is equivalent to 80 mg of free compound, and 122.19 mg of Form A is equivalent to 100 mg of free compound. | | | | | | |

### Experiment 5

### Dissolution of Tablets

Take tablet 2 prepared in Example 1-5 and conduct dissolution testing according to the method essentially consistent with CN113015521A test Example 2 - tablet dissolution (1), under the following conditions:
Dissolution Instrument: RC12AD Tian Da Tian Fa
Dissolution Medium: pH1.2 Buffer
Medium Volume: 900 mL
Dissolution Method: Paddle Method
Medium Temp: 37°C
Rotation Speed: 100 rpm
Sampling Time: 5 min, 10 min, 15 min, 30 min

The dissolution results are shown in Table 5 below.

**Table 5 Dissolution Results of Tablets of Form A**

| Time (min) | Dissolution Rate (%) |
|---|---|
| 5 | 34% |
| 10 | 100% |
| 15 | 100% |
| 30 | 100% |

The results show that the tablet made from the hydrate Form A of lazertinib mesylate has a dissolution rate of 100% in a pH 1.2 solution after 10 minutes; under the same experimental conditions, the tablet made from Form I has a dissolution rate of 100% in a pH 1.2 solution after 5 minutes (CN 113015521 A Test Example 2 - Tablet Dissolution Test (1)), and the dissolution rate of the tablet made from Form A is comparable to that of the tablet made from Form I.

The above examples are the preferred embodiments of the application, but the implementation of the application is not limited to the above examples. Any modifications, alterations, substitutions, combinations, simplifications, or changes made within the scope of the spirit and essence of the present application, which do not depart from the scope of the present application, are considered to be equivalent alternatives and are encompassed within the scope of protection of the present application.

## Claims

1. A hydrate Form A of lazertinib mesylate, with lazertinib having a structure represented by Formula (I): 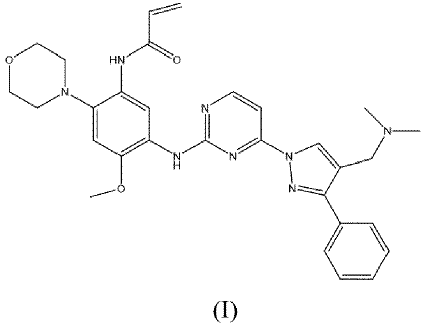 wherein, using Cu-Kα radiation, the X-ray powder diffraction (XRPD) pattern of lazertinib mesylate Form A, is **characterized by** comprising at least three characteristic peaks at 5.68°±0.2°, 11.74±0.2°, 15.60°±0.2°, 21.61°±0.2°, or 22.71°±0.2° 2θ.

2. The hydrate Form A of lazertinib mesylate according to claim 1, wherein the XRPD pattern further comprises at least one characteristic peak at 8.62±0.2°, 10.50°±0.2°, 14.50±0.2°, 16.57°±0.2°, or 17.21±0.2°2θ.

3. The hydrate Form A of lazertinib mesylate according to claim 1 or 2, wherein its XRPD pattern further comprises at least one characteristic peak at 11.24°±0.2°, 13.30±0.2°, 18.17±0.2°, 20.03±0.2°, 24.63°±0.2°, or 28.53±0.2° 2θ.

4. The hydrate Form A of lazertinib mesylate according to any one of claims 1 to 3, wherein its XRPD pattern has characteristic peaks (expressed in 2θ) shown in the following table:
| 2θ±0.2° |
|---|
| 5.68 |
| 8.62 |
| 10.50 |
| 11.24 |
| 11.74 |
| 13.30 |
| 14.51 |
| 15.60 |
| 16.57 |
| 17.21 |
| 17.91 |
| 18.17 |
| 19.55 |
| 20.03 |
| 20.99 |
| 21.61 |
| 22.71 |
| 23.45 |
| 24.63 |
| 25.61 |
| 25.91 |
| 26.57 |
| 27.41 |
| 28.53 |
| 29.61 |

5. The hydrate Form A of lazertinib mesylate according to any one of claims 1 to 4, wherein its XRPD pattern is essentially as depicted in Fig. 1.

6. A process for preparing the hydrate Form A of lazertinib mesylate according to any one of claims 1 to 5, wherein the process comprises any one of the following:
1) dissolve lazertinib and methanesulfonic acid separately in dichloromethane to form a clear solution of lazertinib and a clear solution of methanesulfonic acid; mix the two solutions, precipitate the resulting solid, centrifuge, and dry to obtain the hydrate Form A of lazertinib mesylate; the dissolution of lazertinib is carried out at an elevated temperature, and the molar ratio of lazertinib to methanesulfonic acid is in the range about 1:1 to about 1.2;
preferably, the elevated temperature is 60°C or higher; furthermore, it is in the range of 65-85°C;
preferably, the mixing of the clear solutions involves adding the methanesulfonic acid solution into the lazertinib solution;
preferably, the drying process is conducted through room temperature vacuum drying;
2) form solution 1 by dissolving lazertinib mesylate Form B in solvent 1, then, add solution 1 to solvent 2, stir, precipitate solid material, centrifuge, and dry to obtain the hydrate Form A of lazertinib mesylate. In this process, solvent 1 and solvent 2 are mutually miscible solvents;
preferably, Form B exhibits an X-ray powder diffraction (XRPD) pattern essentially as shown in Fig. 7;
preferably, solvent 1 is selected from trichloromethane;
preferably, solvent 2 is selected from ethyl ether or acetonitrile;
preferably, the volume ratio of solvent 1 to solvent 2 is less than 1:5; more preferably, less than 1:7;
preferably, drying is conducted at room temperature;
3) dry lazertinib mesylate Form B at 40-90°C to obtain the hydrate Form A of lazertinib mesylate;
preferably, the drying temperature is in the range about 40 to about 50°C.

7. A pharmaceutical composition of lazertinib, wherein the composition comprises hydrate Form A of lazertinib mesylate according to any one of the claims 1 to 5, and at least one pharmaceutically acceptable carrier.

8. A formulation prepared from the lazertinib pharmaceutical composition according to claim 7, wherein the formulation includes but not limited to oral solid dosage form, topical formulation, and injectable formulation; preferably tablet.

9. A use of hydrate Form A of lazertinib mesylate according to any one of claims 1 to 5, or the pharmaceutical composition thereof according to claim 7 or 8, in the preparation of drugs for treating protein kinase-mediated diseases.

10. A use of the hydrate Form A of lazertinib mesylate according to any one of claims 1 to 5, or the pharmaceutical composition thereof according to claim 7 or 8, in the preparation of drugs for inhibiting the activity of EGFR with at least one mutation compared to wild-type EGFR.

11. The use according to claim 9, wherein the disease is non-small cell lung cancer or metastatic non-small cell lung cancer.

12. A method for treating a protein kinase-mediated disease, comprises administering to a patient an effective amount of hydrate Form A of lazertinib mesylate according to any one of claims 1 to 5, or the pharmaceutical composition according to claim 7 or 8.

13. A method for inhibiting the activity of EGFR with at least one mutation compared to wild-type EGFR, comprises administering to a patient an effective amount of the hydrate Form A of lazertinib mesylate according to any one of claims 1 to 5, or the pharmaceutical composition according to claim 7 or 8.

14. A combination use of hydrate Form A of lazertinib mesylate according to any one of claims 1 to 5, or its pharmaceutical composition according to claim 7 or 8, with other drugs.
